# EUROPÄISCHE PATENTSCHRIFT

(11) **EP 0 673 920 B1**
(45) Veröffentlichungstag und Bekanntmachung des Hinweises auf die Patenterteilung: **05.11.1997**
(21) Anmeldenummer: 95103937.9
(22) Anmeldetag: 17.03.1995
(51) Int. Cl.: C07C 213/04, C07C 215/08, C07C 215/12

(54) **Verfahren zur Herstellung von Ethanolaminen**
Process for the preparation of ethanolamines
Procédé de préparation d'éthanolamines

(30) Priorität: 26.03.1994 DE 4410610
(43) Veröffentlichungstag der Anmeldung: 27.09.1995
(73) Patentinhaber: BASF AKTIENGESELLSCHAFT, 67056 Ludwigshafen (DE)
(72) Erfinder: Hammer, Hans, D-68219 Mannheim (DE); Reutemann, Werner, D-67240 Bobenheim-Roxheim (DE)

(56) Entgegenhaltungen:
- Keine einschlägigen Dokumente gefunden

## Beschreibung

Die Erfindung betrifft ein Verfahren zur Herstellung von Gemischen aus Mono-, Di- und Triethanolaminen durch Umsetzung von Ammoniak und Ethylenoxid in einem mit einer Kühlung versehenen Rohrreaktor, wobei das Ethylenoxid in einer oder mehreren Teilmengen zugegeben wird.

Es ist allgemein bekannt, daß Ethanolamine durch Reaktion zwischen Ammoniak und Ethylenoxid bei höheren Temperaturen und Drücken hergestellt werden können. Ammoniak wird bevorzugt als wäßrige Lösung eingesetzt, wobei Wasser als Katalysator wirkt. Da es sich um ein System konkurrierender Folgereaktionen handelt, entstehen dabei Mono-, Di- und Triethanolamin nebeneinander.

Aus Chem. Abstr. Vol. 96, 51806 g (1982) ist ein Verfahren zur Herstellung von Alkanolaminen aus Alkylenoxiden mit einem Überschuß einer 5 bis 50 gew.-%igen Ammoniaklösung bei Temperaturen von 30 bis 100°C und Drucken von 3 bis 40 bar bekannt. Dieses Verfahren besteht aus 2 bis 7 adiabatisch betriebenen Rohrreaktoren mit einer Kühlung nach jedem Reaktor. Nachteilig an diesem Verfahren ist der hohe Wassergehalt der Reaktionsmischung, die tiefe Reaktionstemperatur und daraus resultierend lange Verweilzeiten.

Der vorliegenden Erfindung lag daher die Aufgabe zugrunde, den zuvor genannten Nachteilen abzuhelfen.

Demgemäß wurde ein neues und verbessertes Verfahren zur Herstellung von Gemischen aus Mono-, Di- und Triethanolamin durch Umsetzung von Ammoniak und Ethylenoxid in einem mit einer Kühlung versehenen Rohrreaktor bei Temperaturen von 110 bis 150°C und Drucken von 50 bis 120 bar gefunden, welches dadurch gekennzeichnet ist, daß man Ammoniak und Ethylenoxid im Molverhältnis von 1 : 1 bis 100 : 1 und Ammoniak als 60 bis 99,99 %-ige wäßrige Lösung einsetzt und 1 bis 80 % des Ethylenoxids in einer bis zehn Teilmengen im Bereich von 10 bis 70 % der Gesamtlänge des Rohrreaktors zugibt.

Die vorliegende Erfindung läßt sich wie folgt durchführen:

Man kann 20 bis 99 Gew.-%, bevorzugt 40 bis 95 Gew.-%, besonders bevorzugt 50 bis 90 Gew.-% des gesamten Ethylenoxids mit dem gesamten Ammoniak in 60 bis 99,99 %-iger, bevorzugt 65 bis 99 %-iger, besonders bevorzugt 70 bis 95 %-iger wäßriger Lösung vor dem mit einer Kühlung versehenen Rohrreaktor, bevorzugt einem Rohrreaktor mit Doppelmantelkühlung, mischen, bevorzugt auf Temperaturen von 40 bis 70°C vorheizen und einspeisen und das restliche Ethylenoxid von 1 bis 80 Gew.-%, bevorzugt 5 bis 60 Gew.-%, besonders bevorzugt 10 bis 50 Gew.-% in einem oder mehreren, z.B. einem bis zu zehn Anteilen, bevorzugt einem bis acht, besonders bevorzugt einem bis sechs Anteilen verteilt im Bereich von 10 bis 70 %, bevorzugt 20 bis 60 %, besonders bevorzugt 25 bis 50 % der Gesamtlänge des Rohrreaktors zu dem Ammoniak in wäßriger Lösung bzw. dem Reaktionsgemisch zumischen.

Die Reaktionstemperatur im Rohrreaktor beträgt in der Regel maximal 160°C, die Grenzen 110 bis 160°C, bevorzugt 120 bis 150°C und der Reaktionsdruck beträgt in der Regel 50 bis 120 bar, bevorzugt 60 bis 110 bar, besonders bevorzugt 75 bis 100 bar.

Das Molverhältnis von Ammoniak zum Ethylenoxid kann in weiten Grenzen schwanken, betragt in der Regel 1 : 1 bis 100 : 1, bevorzugt 3 : 1 bis 50 : 1, besonders bevorzugt 4 : 1 bis 25 : 1.

Die Umsetzung kann bevorzugt ohne Zusatz eines Ionenaustauschers (als Katalysator) durchgeführt werden.

Man laßt in der Regel bei dem erfindungsgemäßen Verfahren das Ethylenoxid vollständig oder weitgehend abreagieren, bevor der nächste Anteil des Ethylenoxids zu dem Gemisch aus Ammoniak in wäßriger Lösung und dem Reaktionsprodukt (Gemisch aus Monoethanolamin, Diethanolamin, Triethanolamin) gefahren wird.

In wie viele Anteile das Ethylenoxid aufgeteilt und wie groß die einzelnen Anteile sind, ist beliebig und hängt nur von der Konstruktion des Reaktors ab (Beispiel: Aufteilung in 2 Anteile im Gewichtsverhältnis 60 : 40; oder 3 Anteile im Gewichtsverhältnis 60 : 20 : 20, oder 5 Anteile in Gewichtsverhältnis 50 : 20 : 10 : 10 : 10).

Die Steuerung der Anteile der Komponenten Mono-, Di- und Triethanolamin kann in der Regel durch Veränderung des molaren Verhältnisses von Ammoniak zum Ethylenoxid erfolgen. Durch hohen molaren Überschuß von Ammoniak zu Ethylenoxid wird in der Regel ein hoher Anteil von Monoethanolamin gebildet.

Wird der molare Überschuß an Ammoniak bezogen auf Ethylenoxid abgesenkt, so nimmt in der Regel der Anteil von Diethanolamin und Triethanolamin im Reaktionsprodukt auf Kosten des Monoethanolamins deutlich zu.

Die Geschwindigkeit im Rohrreaktor wird in der Regel so gewählt, daß turbulente Strömung herrscht (Vermeidung von Rückvermischung, bessere Wärmeübertragung). Es muß in der Regel verhindert werden, daß Zweiphasigkeit entsteht. Die Maßgabe, daß die Umsetzung in homogener flüssiger Phase vorgenommen werden soll, bedeutet, daß jeweils ein Mindestdruck herrscht, der etwas großer ist, als es dem Dampfdruck des Reaktionsgemisches bei der gewählten Temperatur entspricht. Wesentlich höhere Drucke als die Mindestdrücke bringen keine Nachteile, sind aber auch nicht erforderlich.

Hohe Reaktionstemperaturen über 170°C bringen den Nachteil, daß bei der Aufarbeitung Reinprodukte (Monoethanolamin, Diethanolamin, Triethanolamin) entstehen, die nicht farbstabil sind, d.h. sie verfärben sich bei Lagerung.

Die Reaktion lauft sehr schnell ab. Die erforderliche Verweilzeit zur vollständigen Umsetzung beträgt in der Regel zwischen 1 bis 40 Minuten, wobei die erforderliche Verweilzeit durch Erhöhung der Reaktionstemperatur und der Wasserkonzentration verkürzt werden kann.

Um eine zu starke Erwärmung des Reaktionsproduktes zu verhindern, kann mit Wasser oder Ammoniak verdünnt werden. Die Abtrennung des Ammoniaks oder Wassers erfordert bei der Aufarbeitung jedoch einen großen Energieaufwand. Eine andere Möglichkeit die maximale Reaktionstemperatur zu begrenzen ist, den Durchsatz durch den Reaktor zu vermindern. Damit wird die freiwerdende Reaktionswärme pro Zeiteinheit vermindert. Durch diese Arbeitsweise wird jedoch die Kapazität des Reaktors stark begrenzt.

Vorteilhaft ist es, das Reaktionsprodukt vor der Zugabe eines neuen Anteils an Ethylenoxid soweit abzukühlen (100 bis 140°C), daß die Reaktion zwar sofort anspringt, gleichzeitig die durch die Reaktionswärme erfolgte Temperaturerhöhung nicht dazu führt, daß das Reaktionsprodukt über 150°C erwärmt wird bzw. nicht soweit erwärmt wird, daß Zweiphasigkeit auftritt.

Mit dem erfindungsgemäßen Verfahren ist es möglich, die Reaktion zwischen Ethylenoxid und Ammoniak in einzelne Abschnitte aufzuteilen. Der Energieaustausch wird erleichtert, die maximale Reaktionstemperatur liegt wesentlich niedriger als wenn die gesamte Ethylenoxid-Menge mit dem Ammoniak an einer Stelle gemischt wird. Bei dieser Arbeitsweise ist es nicht erforderlich große Wasseranteile im Einsatzprodukt zu fahren, um die Reaktionswärme in Form von erwärmtem Reaktionsprodukt abzufuhren. Das den Einsatzprodukten zugesetzte Wasser muß aus dem Reaktionsprodukt wieder abdestilliert werden. Mit dem erfindungsgemäßen Verfahren wird deshalb eine erhebliche Energieeinsparung erzielt. Bei dem erfindungsgemäßen Verfahren wird nur soviel Wasser zugesetzt, daß die Verweilzeit der Reaktion ausreicht, um vollständige Umsetzung des eingesetzten Ethylenoxids zu erreichen.

Wärme wird in der Regel auch durch den im Überschuß eingesetzten Ammoniak abgeführt. Bei dem erfindungsgemäßen Verfahren ist es möglich, bei unverändertem Verhältnis Wasser zu Ammoniak mit geringerem Verhältnis Ammoniak zu Ethylenoxid zu arbeiten, ohne daß die maximale Reaktionstemperatur ansteigt. Dies wird ermöglicht, da durch getrennte Einspeisung von Ethylenoxid in mehreren Abschnitten auch die Energie über eine längere Strecke des Rohrreaktors verteilt anfällt. Die weitere Absenkung des Molverhältnisses Ammoniak zu Ethylenoxid erhöht die Flexibilität der Produktion der einzelnen Ethanolamine (Monoethanolamin, Diethanolamin, Triethanolamin).

Durch die Zugabe von Ethylenoxid in mehreren Teilen wird die maximale Reaktionstemperatur abgesenkt. Aus dem Reaktionsprodukt können Reinprodukte (Monoethanolamin, Diethanolamin, Triethanolamin) durch Destillation gewonnen werden, die bei Lagerung ihre Farbe nicht verändern.

Die Absenkung der maximalen Reaktionstemperatur ermöglicht es, den Reaktionsdruck abzusenken, ohne daß Zweiphasigkeit im Reaktor auftritt. Kostenersparnis bei der Investition und geringerer Energieverbrauch für die Kompression der Einsatzstoffe sind das Ergebnis.

Ein weiterer vorteil des erfindungsgmäßen Verfahrens liegt darin, daß durch Aufteilung der zugegeben Ethylenoxid-Menge auf mehrere Abschnitte die Verteilung der produziert Ethanolamine Monoethanolamin : Diethanolamin : Triethanolamin beeinflußt werden kann. Wie oben beschrieben, wird mit Erhöhung des molaren Überschusses von Ammoniak zu Ethylenoxid der Anteil des produzierten Monoethanolamins im Reaktionsgemisch erhöht. Hohe Monoethanolamin-Anteile bedeuten gleichzeitig hohen Ammoniak-Überschuß. Der nicht umgesetzte Ammoniak-Anteil wird in der Regel wieder abdestilliert oder durch Entspannung verdampft. Bei dem erfindungsgemäßen Verfahren ist es möglich, bei unverändertem Verhältnis Ammoniak zu Ethylenoxid ein Ethanolamin-Gemisch mit höherem Monoethanolamin-Anteil zu produzieren. Wird Ammoniak und Ethylenoxid im molaren Verhältnis von 7,6 eingesetzt (Einspeisung von Ethylenoxid an einer Stelle), so wird ein Gemisch erhalten, das zu 52,6 Gew.-% Monoethanolamin, 33,9 Gew.-% Diethanolamin und 13,5 Gew.-% Triethanolamin enthält. Wird bei konstantem molaren Verhältnis von Ammoniak zu Ethylenoxid das erforderliche Ethylenoxid in zwei Teile aufgeteilt (1. Ethylenoxid-Einspeisung: 60 % des Ethylenoxids, 2. Ethylenoxid-Einspeisung: 40 % des Ethylenoxids), so wird ein Gemisch von Ethanolaminen erhalten, das zu 58,7 Gew.-% Monoethanolamin, zu 30,6 Gew.-% aus Diethanolamin und zu 10,7 Gew.-% aus Triethanolamin besteht. Wird das gesamte Ethylenoxid und der Ammoniak an einer Stelle vor dem Reaktor gemischt, so wird der Monoethanolamin-Anteil von 58,7 Gew.-% durch ein molares Verhältnis Ammoniak zu Ethylenoxid von 9,6 erhalten. Durch das erfindungsgemäße Verfahren, kann bei unverändertem molaren Verhältnis von NH₃ : Ethylenoxid der Monoethanolamin-Anteil erhöht werden. Es kann jedoch auch der unveränderte Monoethanolamin-Anteil durch einen geringeren Ammoniaküberschuß erreicht werden. Dies führt zu einer wesentlichen Energieeinsparung, da weniger Ammoniak und Wasser abdestilliert werden müssen.

Monoethanolamin, Diethanolamin und Triethanolamin sind als Lösungsmittel oder als Basen für organische Reaktionen geeignet.

### Beispiele

### Beispiel 1

In einem Rohrreaktor (Länge: 1 500 m, innerer Durchmesser: 125 mm) wird Ethylenoxid und Ammoniak in wäßriger Lösung (87 Gew.-% Ammoniak) bei 90 bar umgesetzt. Die Rohre des Rohrreaktors besitzen jeweils einen Doppelmantel. Die ersten 100 m des Rohrreaktors können über den Doppelmantel aufgeheizt werden. Die weiteren 1 400 m werden über den Doppelmantel gekühlt. Die Kühlung kann so eingestellt werden, daß eine vorgeschriebene maximale Reaktionstemperatur nicht überschritten wird.

Mit geeigneten Pumpen wird der Ammoniak in wäßriger Lösung auf den Reaktionsdruck hochgepreßt. Ethylenoxid wird ebenfalls auf den Reaktionsdruck gepreßt. Beide Ströme werden direkt vor dem Reaktor gemischt. Es besteht die Möglichkeit, Teilströme des Ethylenoxids an verschiedenen Stellen, die auf der Länge des Reaktors verteilt sind, in den Reaktor zu fahren. Am Ende des Reaktors wird der Reaktionsauftrag entspannt. Der nicht umgesetzte Ammoniak und das Wasser werden abdestilliert. Das verbleibende Ethanolamin-Gemisch wird üblicherweise destillativ zu den Produkten Monoethanolamin, Diethanolamin und Triethanolamin aufgearbeitet.

Es wurden 10 t/h NH₃ in wäßriger Lösung (NH₃-Gehalt = 87 Gew.-%) mit Ethylenoxid bei 20°C gemischt und in den Reaktor gefahren. Ethylenoxid wurde aufgesplittet. Ein Teil wurde direkt vor dem Reaktor mit NH₃ in wäßriger Lösung gemischt und in den Reaktor gefahren (1. Einspeisung). Der andere Teil des Ethylenoxids wurde etwa 500 m nach dem Reaktoranfang in den Reaktor gefahren (2. Einspeisung)
- Versuch 1:: 1. Einspeisung = 5 900 kg/h
2. Einspeisung = 0 kg/h
- Versuch 2:: 1. Einspeisung = 5 300 kg/h
2. Einspeisung = 600 kg/h
- Versuch 3:: 1. Einspeisung = 4 700 kg/h
2. Einspeisung = 1 200 kg/h
- Versuch 4:: 1. Einspeisung = 4 100 kg/h
2. Einspeisung = 1 800 kg/h
- Versuch 5:: 1. Einspeisung = 3 500 kg/h
2. Einspeisung = 2 400 kg/h

**Tabelle A**

| | Versuch 1 | Versuch 2 | Versuch 3 | Versuch 4 | Versuch 5 |
|---|---|---|---|---|---|
| max. Reaktionstemperatur | 147°C | 144°C | 141°C | 139°C | 136°C |

| Gewichts-% im Ethanolamin-Gemisch | | | | | |
|---|---|---|---|---|---|
| Monoethanolamin | 52,7 | 54,6 | 55,8 | 57,1 | 58,7 |
| Diethanolamin | 33,9 | 32,9 | 32,6 | 31,6 | 30,6 |
| Triethanolamin | 13,4 | 12,5 | 11,6 | 11,3 | 10,7 |

### Beispiel 2

Reaktor und Verfahrensweise wie in Bespiel 1. Es wurden 26 t/h NH₃ in wäßriger Lösung (NH₃-Gehalt = 87 Gew.-%) mit Ethylenoxid bei 20°C gemischt und in den Reaktor gefahren. Ethylenoxid wurde aufgesplittet. Ein Teil wurde direkt vor dem Reaktor mit NH₃ in wäßriger Lösung gemischt und in den Reaktor gefahren (1. Einspeisung). Der andere Teil des Ethylenoxids wurde etwa 500 m nach dem Reaktoranfang in den Reaktor gefahren (2. Einspeisung).
- Versuch 1:: 1. Einspeisung = 6 500 kg/h
2. Einspeisung = 0 kg/h
- Versuch 2:: 1. Einspeisung = 5 850 kg/h
2. Einspeisung = 650 kg/h
- Versuch 3:: 1. Einspeisung = 5 200 kg/h
2. Einspeisung = 1 300 kg/h
- Versuch 4:: 1. Einspeisung = 4 550 kg/h
2. Einspeisung = 1 950 kg/h
- Versuch 5:: 1. Einspeisung = 3 900 kg/h
2. Einspeisung = 2 600 kg/h

**Tabelle B**

| | Versuch 1 | Versuch 2 | Versuch 3 | Versuch 4 | Versuch 5 |
|---|---|---|---|---|---|
| max. Reaktionstemperatur | 146°C | 144°C | 141°C | 139°C | 136°C |

| Gewichts-% im Ethanolamin-Gemisch | | | | | |
|---|---|---|---|---|---|
| Monoethanolamin | 58,1 | 59,3 | 60,6 | 61,8 | 63,0 |
| Diethanolamin | 31,6 | 30,8 | 30,0 | 29,8 | 29,6 |
| Triethanolamin | 10,3 | 9,9 | 9,4 | 8,4 | 7,4 |

## Patentansprüche

1. Verfahren zur Herstellung von Gemischen aus Mono-, Di- und Triethanolamin durch Umsetzung von Ammoniak und Ethylenoxid in einem mit einer Kühlung versehenen Rohrreaktor bei Temperaturen von 110 bis 160°C und Drücken von 50 bis 120 bar, dadurch gekennzeichnet, daß man Ammoniak und Ethylenoxid im Molverhältnis von 1 : 1 bis 100 : 1 und Ammoniak als 60 bis 99,99 %-ige wäßrige Lösung einsetzt und 1 bis 80 % des Ethylenoxids in einer bis zehn Teilmengen im Bereich von 10 bis 70 % der Gesamtlänge des Rohrreaktors zugibt.

2. Verfahren zur Herstellung von Gemischen aus Mono-, Di- und Triethanolamin nach Anspruch 1, dadurch gekennzeichnet, daß man 5 bis 60 % des Ethylenoxids in einer bis acht Teilmengen im Bereich von 20 bis 60 % der Gesamtlänge des Rohrreaktors zugibt.

3. Verfahren zur Herstellung von Gemischen aus Mono-, Di- und Triethanolamin nach Anspruch 1, dadurch gekennzeichnet, daß man 10 bis 50 % des Ethylenoxids in einer bis sechs Teilmengen im Bereich von 25 bis 50 % der Gesamtlänge des Rohrreaktors zugibt.

4. Verfahren zur Herstellung von Gemischen aus Mono-, Di- und Triethanolamin nach Anspruch 1, dadurch gekennzeichnet, daß man die Umsetzung bei Temperaturen von 120 bis 150°C durchführt.

5. Verfahren zur Herstellung von Gemischen aus Mono-, Di- und Triethanolamin nach Anspruch 1, dadurch gekennzeichnet, daß man die Umsetzung bei Drücken von 60 bis 110 bar durchführt.

6. Verfahren zur Herstellung von Gemischen aus Mono-, Di- und Triethanolamin nach Anspruch 1, dadurch gekennzeichnet, daß man die Umsetzung bei Drücken von 75 bis 100 bar durchführt.

7. Verfahren zur Herstellung von Gemischen aus Mono-, Di- und Triethanolamin nach Anspruch 1, dadurch gekennzeichnet, daß man Ammoniak als 65 bis 99 %-ige wäßrige Lösung einsetzt.

8. Verfahren zur Herstellung von Gemischen aus Mono-, Di- und Triethanolamin nach Anspruch 1, dadurch gekennzeichnet, daß man Ammoniak als 70 bis 95 %-ige wäßrige Lösung einsetzt.

9. Verfahren zur Herstellung von Gemischen aus Mono-, Di- und Triethanolamin nach Anspruch 1, dadurch gekennzeichnet, daß man Ammoniak als 75 bis 90 %-ige wäßrige Lösung einsetzt.

10. Verfahren zur Herstellung von Gemischen aus Mono-, Di- und Triethanolamin nach Anspruch 1, dadurch gekennzeichnet, daß man Ammoniak und Ethylenoxid im Molverhältnis von 3 : 1 bis 50 : 1 einsetzt.

## Claims

1. A process for producing mixtures of mono-, di- and triethanolamine by reacting ammonia and ethylene oxide in a cooled tubular reactor at temperatures from 110 to 160°C and pressures from 50 to 120 bar, which comprises using ammonia and ethylene oxide in a molar ratio from 1:1 to 100:1 and ammonia in the form of a from 60 to 99.99% strength aqueous solution and adding from 1 to 80% of the ethylene oxide in from one to ten aliquots over from 10 to 70% of the total length of the tubular reactor.

2. A process for producing mixtures of mono-, di- and triethanolamine as claimed in claim 1, wherein from 5 to 60% of the ethylene oxide is added in from one to eight aliquots over from 20 to 60% of the total length of the tubular reactor.

3. A process for producing mixtures of mono-, di- and triethanolamine as claimed in claim 1, wherein from 10 to 50% of the ethylene oxide is added in from one to six aliquots over from 25 to 50% of the total length of the tubular reactor.

4. A process for producing mixtures of mono-, di- and triethanolamine as claimed in claim 1, wherein the reaction is carried out at temperatures from 120 to 150°C.

5. A process for producing mixtures of mono-, di- and triethanolamine as claimed in claim 1, wherein the reaction is carried out at pressures from 60 to 110 bar.

6. A process for producing mixtures of mono-, di- and triethanolamine as claimed in claim 1, wherein the reaction is carried out at pressures from 75 to 100 bar.

7. A process for producing mixtures of mono-, di- and triethanolamine as claimed in claim 1, wherein ammonia is used as a from 65 to 99% strength aqueous solution.

8. A process for producing mixtures of mono-, di- and triethanolamine as claimed in claim 1, wherein ammonia is used as a from 70 to 95% strength aqueous solution.

9. A process for producing mixtures of mono-, di- and triethanolamine as claimed in claim 1, wherein ammonia is used as a from 75 to 90% strength aqueous solution.

10. A process for producing mixtures of mono-, di- and triethanolamine as claimed in claim 1, wherein ammonia and ethylene oxide are used in a molar ratio from 3:1 to 50:1.

## Revendications

1. Procédé de préparation de mélanges de mono-, di- et tri-éthanolamines par réaction de l'ammoniac et de l'oxyde d'éthylène dans un réacteur tubulaire équipé d'un dispositif de refroidissement à des températures de 110 à 160° C et des pressions de 50 à 120 bar, caractérisé par le fait que l'on met en oeuvre l'ammoniac et l'oxyde d'éthylène à un rapport molaire de 1 : 1 à 100 : 1 et l'ammoniac à l'état de solution aqueuse à une concentration de 60 à 99,99 %, et on introduit de 1 à 80 % de l'oxyde d'éthylène en une à dix portions dans la région de 10 à 70 % de la longueur totale du réacteur tubulaire.

2. Procédé de préparation de mélanges de mono-, di- et tri-éthanolamines selon la revendication 1, caractérisé par le fait que l'on introduit de 5 à 60 % de l'oxyde d'éthylène en une à huit portions dans la région de 20 à 60 % de la longueur totale du réacteur tubulaire.

3. Procédé de préparation de mélanges de mono-, di- et tri-éthanolamines selon la revendication 1, caractérisé par le fait que l'on introduit de 10 à 50 % de l'oxyde d'éthylène en une à six portions dans la région de 25 à 50 % de la longueur totale du réacteur tubulaire.

4. Procédé de préparation de mélanges de mono-, di- et tri-éthanolamines selon la revendication 1, caractérisé par le fait que l'on effectue la réaction à des températures de 120 à 150° C.

5. Procédé de préparation de mélanges de mono-, di- et tri-éthanolamines selon la revendication 1, caractérisé par le fait que l'on effectue la réaction à des pressions de 60 à 110 bar.

6. Procédé de préparation de mélanges de mono-, di- et tri-éthanolamines selon la revendication 1, caractérisé par le fait que l'on effectue la réaction sous des pressions de 75 à 100 bar.

7. Procédé de préparation de mélanges de mono-, di- et tri-éthanolamines selon la revendication 1, caractérisé par le fait que l'on met en oeuvre l'ammoniac à l'état de solution aqueuse à une concentration de 65 à 99 %.

8. Procédé de préparation de mélanges de mono-, di- et tri-éthanolamines selon la revendication 1, caractérisé par le fait que l'on met en oeuvre l'ammoniac à l'état de solution aqueuse à une concentration de 70 à 95 %.

9. Procédé de préparation de mélanges de mono-, di- et tri-éthanolamines selon la revendication 1, caractérisé par le fait que l'on met en oeuvre l'ammoniac à l'état de solution aqueuse à une concentration de 75 à 90 %.

10. Procédé de préparation de mélanges de mono-, di- et tri-éthanolamines selon la revendication 1, caractérisé par le fait que l'on met en oeuvre l'ammoniac et l'oxyde d'éthylène a un rapport molaire de 3 : 1 à 50 : 1.
